# FASCICULE DE BREVET EUROPEEN

(11) **EP 2 548 585 B1**
(45) Date de publication et mention de la délivrance du brevet: **23.08.2017**
(21) Numéro de dépôt: 12176897.2
(22) Date de dépôt: 18.07.2012
(51) Int. Cl.: A61L 2/18, A01N 47/44, A01N 33/12

(54) **Composition nettoyante et/ou désinfectante**
Reinigungs- und/oder Desinfektionszusammensetzung
Cleaning and/or disinfecting composition

(30) Priorité: 19.07.2011 FR 1102238
(43) Date de publication de la demande: 23.01.2013
(73) Titulaire: Laboratoires Anios, 59260 Lille-Hellemmes (FR)
(72) Inventeur: Letartre, Bertrand, 59260 HELLEMMES (FR); Criquelion, Jacques, 59260 HELLEMMES (FR); Rauwel, Gaétan, 59260 HELLEMMES (FR)
(74) Mandataire: Bureau Duthoit Legros Associés

(56) Documents cités:
- WO-A1-2012/160386
- DE-A1- 3 334 555
- JP-A- 3 215 411
- US-A- 4 642 234
- US-A- 5 453 435
- US-A1- 2004 265 246

## Description

La présente invention concerne l'utilisation d'une composition nettoyante et/ou désinfectante concentrée pour le nettoyage et/ou la désinfection des surfaces et des matériels.

La désinfection est un procédé bien connu destiné à détruire des agents pathogènes tels que les bactéries, les virus, les levures, les moisissures, les champignons ou autres. La désinfection de surfaces ou de matériels permet donc d'éviter la transmission de ces agents pathogènes à l'Homme. Elle est effectuée couramment par tout un chacun et de manière systématique en milieu hospitalier, dans le secteur médical ou paramédical, vétérinaire, dans le domaine de la pharmacie, des cosmétiques et de l'agroalimentaire. Plus particulièrement, la désinfection de surfaces, d'instruments et de matériels est obligatoire en milieu médical ou vétérinaire, notamment dans les cliniques et les hôpitaux.

Il existe de nombreuses compositions nettoyantes et/ou désinfectantes utilisables directement ou à diluer dans de l'eau qui sont employées en milieu médical. Parmi celles-ci, les compositions comprenant des agents antimicrobiens de la famille des biguanides tels que la chlorhexidine ou ses dérivés sont d'un usage très répandu. Ces compositions peuvent comprendre en outre un ou plusieurs autres agents antimicrobiens. Une composition à base de sels de chlorhexidine et de thiosulfate de sodium est par exemple connue par le document US-A-4642234 et utilisée pour la désinfection de lentilles de contact. La chlorhexidine est notamment un antiseptique aux effets bactéricides ou bactériostatiques sur les bactéries à gram positif ou négatif. Il est donc avantageux de l'utiliser en raison de son large spectre d'action antibactérien mais aussi pour son profil toxicologique et éco-toxicologique favorable à faible dose. En effet, des solutions aqueuses diluées de chlorhexidine sont utilisées comme antiseptiques cutanés ou bucco-dentaires.

Dans les milieux hospitaliers, l'eau chaude courante utilisée est préalablement traitée, le plus souvent par ajout de dérivés de chlore ou d'eau de javel, cela permet notamment la prévention du risque de légionellose. Cette eau dite chlorée contient donc des ions hypochlorite (ClO⁻) et de l'acide hypochloreux (HOCl).

Il a été récemment observé que la dilution de la composition ANIOS ^{®} réf. 1916 (qui comprend de la chlorhexidine) avec de l'eau courante javellisée en milieu hospitalier donnait une coloration jaune à la solution obtenue.

Par ailleurs, l'hypochlorite de sodium est en solution diluée dans l'eau un antiseptique buccal utilisé en médecine dentaire au même titre que la chlorhexidine. Toutefois, l'utilisation concomitante d'hypochlorite de sodium et de chlorhexidine entraîne, selon les quantités utilisées, la formation d'un précipité marron (Zehnder M., JOE 2006 ; 37, 38-41) ou l'apparition d'une coloration jaune.

Ce précipité marron et cette couleur jaune sont en partie attribués à une réaction d'oxydation de la chlorhexidine par les ions hypochlorite (Basrani et al., JOE 2007 ; 33, 966-969).

Par ailleurs, la demanderesse a maintenant observé qu'une solution comprenant de chlorhexidine et des ions hypochlorite se dégradait rapidement en plusieurs sous produits.

La coloration jaune des solutions désinfectantes à base de chlorhexidine n'est pas acceptable pour l'utilisateur, elle donne un aspect « salissure » et risque de colorer et détériorer les surfaces sur lesquelles elles sont employées.

De plus, les solutions diluées de composition comprenant de la chlorhexidine et des ions hypochlorite sont instables, la chlorhexidine se dégrade rapidement. En conséquence, la capacité antimicrobienne de telles solutions est fortement altérée et ne répond certainement plus aux exigences et besoins qui sont nécessaires en milieu médical.

Pour ces raisons, l'utilisation de chlorhexidine en présence d'ions hypochlorite, et donc d'eau préalablement javellisée ou chlorée, est délicate à mettre en oeuvre, notamment en milieu hospitalier et peut de surcroît présenter un risque sanitaire. Il y a donc un réel besoin de trouver des compositions comprenant de la chlorhexidine ou un de ses dérivés qui puissent être diluées dans de l'eau préalablement javellisée sans que la chlorhexidine ne soit dégradée.

Il est du mérite de la demanderesse d'avoir mis au point une composition comprenant de la chlorhexidine ou un de ses dérivés qui, en contact avec des ions hypochlorite, ne se dégrade pas, c'est-à-dire qu'il n'y a pas ou très peu de dégradation de la chlorhexidine.

La présente invention concerne l'utilisation d'une composition nettoyante et/ou désinfectante comprenant de la chlorhexidine ou un sel de chlorhexidine, caractérisée en ce qu'elle comprend en outre un agent réducteur choisi parmi le groupe constitué par les thiosulfates minéraux, pour le nettoyage et/ou la désinfection de surfaces dures choisies parmi les sols, les murs, les carrelages, plans de travail, tables, portes, placards et chariots, ou de matériels à usage médical, paramédical, vétérinaire, pharmaceutique ou agro-alimentaire choisis parmi les instruments chirurgicaux, instruments médicaux comprenant les endoscopes, tubes et tuyaux, bassines, la verrerie et les équipements de laboratoire.

Le sel de chlorhexidine est choisi parmi le gluconate de chlorhexidine, le digluconate de chlorhexidine, l'acétate de chlorhexidine, le diacétate de chlorhexidine, le chlorhydrate de chlorhexidine et le dichlorhydrate de chlorhexidine, et leurs hydrates.

L'expression « thiosulfates minéraux » selon l'invention, désigne, sans s'y limiter, les thiosulfates alcalins, notamment le thiosulfate de lithium, le thiosulfate de sodium, le thiosulfate de potassium, les thiosulfates alcalino-terreux, notamment le thiosulfate de calcium, et leurs dérivés solvates, en particulier les hydrates.

Avantageusement, la composition comprend un sel de chlorhexidine, plus particulièrement et préférentiellement du digluconate de chlorhexidine.

La quantité de chlorhexidine ou de sel de chlorhexidine est comprise entre 1 et 50 mg/g de composition désinfectante, préférentiellement entre 1 et 25 mg/g, plus préférentiellement d'environ 5 mg/g.

Avantageusement, l'agent réducteur est choisi parmi le groupe constitué par le thiosulfate de sodium, le thiosulfate de potassium, le thiosulfate de calcium. Le thiosulfate de sodium est préféré car il procure une plus grande stabilité à la composition obtenue.

La quantité d'agent réducteur est comprise entre 0.5 et 50 mg/g de composition désinfectante, préférentiellement entre 2 et 6 mg/g, plus préférentiellement d'environ 4 mg/g.

Dans un mode de réalisation, la quantité de chlorhexidine ou de sel de chlorhexidine est comprise entre 1 et 50 mg/g de composition désinfectante et la quantité d'agent réducteur est comprise entre 0.5 et 50 mg/g de composition désinfectante. De préférence, la quantité de chlorhexidine ou de sel de chlorhexidine est comprise entre 1 et 25 mg/g de composition désinfectante et la quantité d'agent réducteur est comprise entre 2 et 6 mg/g de composition désinfectante; préférentiellement la quantité de chlorhexidine ou de sel de chlorhexidine est d'environ 5 mg/g de composition désinfectante et la quantité d'agent réducteur est d'environ 4 mg/g de composition désinfectante.

La composition utilisée selon l'invention se présente généralement sous la forme d'une solution aqueuse. On utilisera avantageusement pour leur préparation de l'eau distillée ou déminéralisée, ayant éventuellement subie une filtration sur une membrane de faible porosité, de préférence de 0.2 µm.

Dans un mode de réalisation, la composition utilisée selon l'invention est caractérisée en ce qu'elle se présente sous forme d'un concentré liquide comprenant de 50 à 70 %, préférentiellement de 55 à 65%, plus préférentiellement environ 60 %, en poids d'eau rapporté au poids total de la composition.

Dans un autre mode de réalisation, la composition utilisée selon l'invention comprend de 99 à 99.95%, préférentiellement de 99.5 à 99.95 %, plus préférentiellement d'environ 99.9 %, en volume d'eau rapporté au volume total de la composition.

De telles compositions peuvent être obtenues par dilution de la composition liquide concentrée décrite plus haut. Les solutions obtenues par dilution avec de l'eau courante chlorée utilisée en milieu hospitalier sont limpides et ne prennent pas une coloration jaune. Elles peuvent ensuite être appliquées sur des surfaces ou des matériels, elles possèdent les propriétés nettoyantes et désinfectantes souhaitées et ne nécessitent pas de rinçage des surfaces ou matériels sur lesquels elles ont été utilisées.

Il est également avantageux d'ajuster le pH de la composition utilisée selon l'invention par ajout d'un acide minéral ou d'une base minérale selon le pH souhaité. De préférence, le pH de la composition selon l'invention est acide (inférieur à 7) ou neutre (environ égal à 7). En général, le pH des compositions contenant des sels acides de chlorhexidine est acide ou légèrement acide, l'ajout d'une base telle que l'hydroxyde de sodium permet d'ajuster le pH autour de 7.

Il est avantageux d'ajouter à la composition utilisée selon l'invention un ou plusieurs agent(s) antimicrobien(s) supplémentaire(s) en combinaison avec la chlorhexidine. Cela permet d'élargir le spectre biocide de la composition.

La composition utilisée selon l'invention peut en outre comprendre un ou plusieurs agent(s) antimicrobien(s) supplémentaire(s) choisi(s) parmi le groupe constitué par le polyhexaméthylène biguanide (PHMB), le chlorhydrate de polyhexaméthylène biguanide (PHMB-HCl), polyaminopropylbiguanide (PAPB), le chlorure de benzalkonium, le chlorure de benzethonium, le chlorure de methylbenzethonium, le chlorure de cetalkonium, le chlorure de cetylpyridinium, le chlorure de cetrimonium, le chlorure de didécyldiméthylammonium, le carbonate de didécyldiméthyl ammonium, le propionate de didécylméthylpolyoxyéthyl ammonium, la laurylamine, la N-(3-aminopropyl)-N-dodécylpropane-1,3-diamine, la tétra-acétyl éthylène diamine, le phénoxy éthanol et le phénoxy propanol, préférentiellement le polyhexaméthylène biguanide (PHMB), le chlorhydrate de polyhexaméthylène biguanide (PHMB-HCl), le chlorure de didécyldiméthylammonium et la N-(3-aminopropyl)-N-dodécylpropane-1,3-diamine.

Dans un mode de réalisation, la composition utilisée selon l'invention comprend du polyhexaméthylène biguanide (PHMB) ou du chlorhydrate de polyhexaméthylène biguanide (PHMB-HCl) et du chlorure de didécyldiméthylammonium, préférentiellement du chlorhydrate de polyhexaméthylène biguanide (PHMB-HCl) et du chlorure de didécyldiméthylammonium, en tant qu'agents microbiens supplémentaires.

Avantageusement, la quantité de PHMB-HCl contenue dans la composition utilisée selon l'invention est comprise entre 0.1 et 50 mg/g de composition désinfectante, préférentiellement entre 0.2 et 0.3 mg/g, plus préférentiellement d'environ 0.24 mg/g; la quantité de chlorure de didécyldiméthylammonium est elle comprise entre 1 et 100 mg/g, préférentiellement entre 80 et 85 mg/g, plus préférentiellement d'environ 82 mg/g.

Dans un autre mode de réalisation, la composition utilisée selon l'invention comprend de la N-(3-aminopropyl)-N-dodécylpropane-1,3-diamine et du chlorure de didécyldiméthylammonium en tant qu'agents microbiens supplémentaires.

Avantageusement, la quantité de N-(3-aminopropyl)-N-dodécylpropane-1,3-diamine) contenue dans la composition utilisée selon l'invention est comprise entre 20 et 80 mg/g de composition désinfectante, préférentiellement entre 40 et 60 mg/g, plus préférentiellement d'environ 51 mg/g; la quantité de chlorure de didécyldiméthylammonium est elle comprise entre 10 et 50 mg/g, préférentiellement entre 20 et 30 mg/g, plus préférentiellement d'environ 25 mg/g.

Généralement, la composition utilisée selon l'invention comprend en outre un ou plusieurs excipient(s) choisi(s) dans le groupe constitué par les émulsifiants, les tensioactifs de type non-ioniques, cationiques ou encore à caractère amphotère, les hydrotropes, les tiers-solvants de type polyol tels que le glycérol.

L'expression « hydrotrope » selon l'invention, désigne, sans s'y limiter, le *para*-toluènesulfonate de sodium, le xylènesulfonate de sodium.

Dans un mode de réalisation préféré, la composition utilisée selon l'invention comprend :
- entre 1 et 50 mg/g de composition désinfectante d'un sel de chlorhexidine, préférentiellement environ 5 mg/g de digluconate de chlorhexidine, et
- entre 0.5 et 50 mg/g de composition désinfectante d'un thiosulfate minéral, préférentiellement environ 4 mg/g d'un thiosulfate alcalin, notamment le thiosulfate de sodium, et
- entre 1 et 100 mg/g de composition désinfectante, préférentiellement environ 82 mg/g, de chlorure de didécyldiméthylammonium, et
- entre 0.1 et 50 mg/g de composition désinfectante, préférentiellement environ 0.24 mg/g, de polyhexaméthylène biguanide ou de chlorhydrate de polyhexaméthylène biguanide, de préférence de chlorhydrate de polyhexaméthylène biguanide, et
- éventuellement un ou plusieurs excipients(s).

La composition utilisée selon l'invention peut en outre comprendre d'autres agents couramment utilisés dans le domaine des désinfectants tels qu'un ou plusieurs parfum(s), de préférence non-allergène(s) et non-sensibilisant(s), et/ou un ou plusieurs colorant(s).

En effet, il est courant d'ajouter un colorant à ces compositions afin de les distinguer clairement les unes des autres et éviter qu'elles ne soient confondues entre elles par l'utilisateur.

La composition utilisée selon l'invention est caractérisée en ce qu'elle se présente sous forme d'un liquide, éventuellement coloré, qui s'écoule et se transvase facilement et qui préférentiellement se mélange / solubilise instantanément en dilution dans l'eau.

Ce concentré liquide est destiné à être dilué dans de l'eau, par exemple de l'eau du robinet, de l'eau préalablement traitée par ajout de chlore, d'eau de javel ou autre produit désinfectant, de l'eau distillée, déminéralisée ou purifiée. La solution diluée obtenue est limpide et éventuellement légèrement colorée.

### EXEMPLES

Les exemples ci-après sont donnés afin d'illustrer l'invention mais ne sont en rien limitatifs quant à la portée de l'invention et des revendications.

Les abréviations suivantes sont utilisées : BSA : albumine de sérum bovin, cfu : unité formant une colonie, CHLX : chlorhexidine, g : gramme(s) ; h : heure(s), kg : kilogramme(s), L : litre(s), m : mètre(s), mg : milligramme(s), mL : millilitre(s), mm : millimètre(s), µm : micromètre(s), min. : minute(s), TFA : acide trifluoroacétique,

### Description des figures:

La figure 1a montre le chromatogramme de la solution 1 au temps t=0 h.
La figure 1b montre le chromatogramme de la solution 1 au temps t=8 h.
La figure 1c montre le chromatogramme de la solution 1 au temps t=24 h.
La figure 2a montre le chromatogramme de la solution 2 au temps t=0 h.
La figure 2b montre le chromatogramme de la solution 2 au temps t=8 h.
La figure 2c montre le chromatogramme de la solution 2 au temps t=24 h.
La figure 3a montre le chromatogramme de la solution 3 au temps t=0 h.
La figure 3b montre le chromatogramme de la solution 3 au temps t=8 h.
La figure 3c montre le chromatogramme de la solution 3 au temps t=24 h.
La figure 4a montre le chromatogramme de la solution 4 au temps t=0 h.
La figure 4b montre le chromatogramme de la solution 4 au temps t=8 h.
La figure 4c montre le chromatogramme de la solution 4 au temps t=24 h.

### Méthode analytique:

Les spectres HPLC ont été obtenus sur un système intégré Alliance commercialisé par WATERS en utilisant une détection UV. Ce système intégré comprend un module de séparation 2690 et un détecteur à barrettes de diodes 996 réglé sur une longueur d'onde de 260 nm. La colonne utilisée est une colonne HyPURITY (HYPERSIL) contenant une phase stationnaire C18 de 5 µm, les dimensions de la colonne sont de 4.6 x 250mm.

L'éluent est un mélange en volumes de 60% de solution A (0.1% v/v TFA dans de l'eau) et de 40% solution B (0.08% v/v de TFA dans de l'acétonitrile). L'analyse a été réalisée avec cet éluent en mode isocratique avec un débit de 1 mL/min et une température de colonne de 30 °C. Le volume d'injection des solutions analysées est de 20 µL.

Dans ces conditions, la chlorhexidine a un temps de rétention compris entre 5 et 5.5 min.

### Exemple 1 : préparation de compositions utilisées selon l'invention.

Les compositions A et B ont été préparées par ajout de 0.4 % en poids d'un agent réducteur à une solution non diluée d'ANIOS ^{®}1916 (commercialisée par la demanderesse).

Les solutions obtenues comprennent donc les produits suivants :
- digluconate de chlorhexidine 5 mg/g,
- chlorure de didecyldimethylammonium 82 mg/g,
- chlorhydrate de polyhexaméthylène biguanide 0.24 mg/g,
- agent réducteur 4 mg/g,
- colorants, parfum, eau déminéralisée filtrée sur membrane 0.2 µm,
- tensioactifs non ioniques ou amphotériques ;

**Tableau 1 : Agents réducteurs compris dans les solutions A et B.**

| **Composition** | **Agent réducteur** |
|---|---|
| **A** | Thiosulfate de sodium |
| **B** | Acide ascorbique |

Ces compositions A et B ont ensuite été diluées à 0.25 % en poids dans de l'eau de réseau pour donner respectivement les solutions A et B.

La solution A est limpide et de couleur vert pâle ce qui est identique à une solution obtenue selon la même dilution à partir d'ANIOS®1916

La solution B en revanche était un peu trouble avec la formation d'un précipité et l'apparition d'une couleur jaune pâle.

En raison des aspects physiques obtenus, le thiosulfate de sodium est préféré à l'acide ascorbique même si ce dernier permet également la stabilisation de solutions de chlorhexidine contenant de l'hypochlorite de sodium.

### Exemple 2 : étude de stabilité d'une composition selon l'invention en solution aqueuse chlorée.

Les 4 solutions suivantes ont été préparées par dilution de 5 g de composition A ou d'ANIOS ®1916 dans 2 kg d'eau courante, avec ou sans ajout d'hypochlorite de sodium :

**Tableau 2 : Composition des solutions 1, 2, 3 et 4.**

| **Solution** | **Quantité de Na₂S₂O₃ (Thiosulfate de sodium)** | **Quantité de NaOCl (Hypochlorite de sodium)** |
|---|---|---|
| 1 | 0 | 0 |
| 2 | 0 | 2 ppm |
| 3 | 0.4% (p/p) | 0 |
| 4 | 0.4% (p/p) | 2 ppm |

| | | |
|---|---|---|
| % (p/p) : pourcentage en poids ppm : partie par million | | |

Les 4 solutions ont été maintenues à température ambiante pendant 24 h. Aux temps t=0, 1 h, 8 h et 24 h, un aliquot de chacune des solutions a été prélevé puis analysé par HPLC. Les quantités de chlorhexidine (quantité de chlorhexidine / quantité de solution en poids) sont exprimées en ppm (parties par million).

Les résultats sont répertoriés dans le tableau 3 ci-dessous :

**Tableau 3 : Résultats de la stabilité des solutions 1, 2, 3 et 4 en présence d'hypochlorite de sodium.**

| **Identification des solutions** | **Dilution dans eau de réseau en % (p/p)** | **Quantité théorique de CHLX dans la solution en ppm** | **Quantité de CHLX à T=0 * en ppm** | **Quantité de CHLX à T =1h * en ppm** | **Quantité de CHLX à T =8h * en ppm** | **Quantité de CHLX à T =24h * en ppm** |
|---|---|---|---|---|---|---|
| **Solution 1** | 0,25 % | 62,5 | 67,5 | 70,6 | 70 | 68,5 |
| d'ANIOS ^{®}1916 (Lot N011.13S) | | | Solutions limpides de couleur vert pâle | | | |
| **Solution 2** | 0,25 % | 62,5 | 23,8 | 22,6 | 23,7 | 25,2 |
| d'ANIOS ^{®}1916 (Lot N011.13S) + 2 ppm de NaOCl | | | Solution marron clair | Solution marron clair | Solution marron | Solution marron clair |
| **Solution 3** | 0,25 % | 62,5 | 74,0 | 79,0 | 80,0 | 73,3 |
| d'ANIOS ^{®}1916 (Lot N011.13S) + 0,4 % Na₂S₂O₃ | | | Solutions limpides de couleur vert pâle | | | |
| **Solution 4** | 0,25 % | 62,5 | 66,4 | 66,5 | 77,8 | 70,1 |
| d'ANIOS ^{®}1916 (Lot N011.13S) + 0,4 % Na₂S₂O₃ + 2 ppm de NaOCl | | | Solutions limpides de couleur vert pâle | | | |

| | | | | | | |
|---|---|---|---|---|---|---|
| * : déterminé par HPLC | | | | | | |

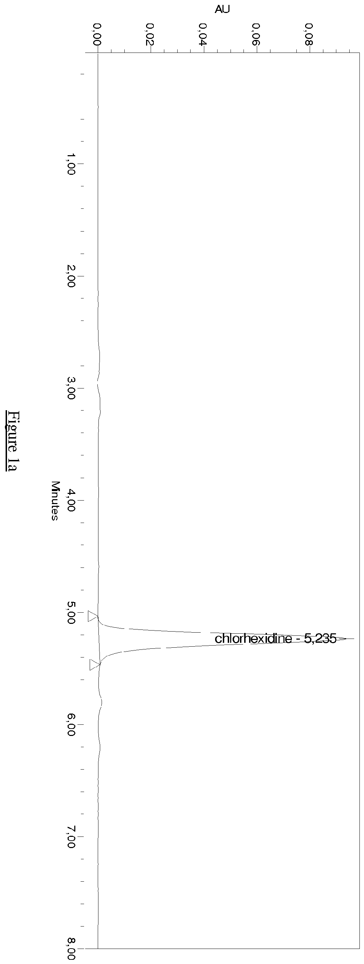

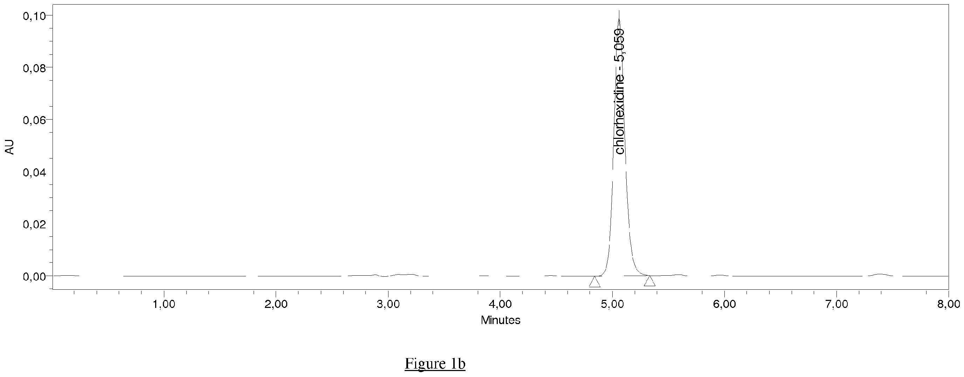

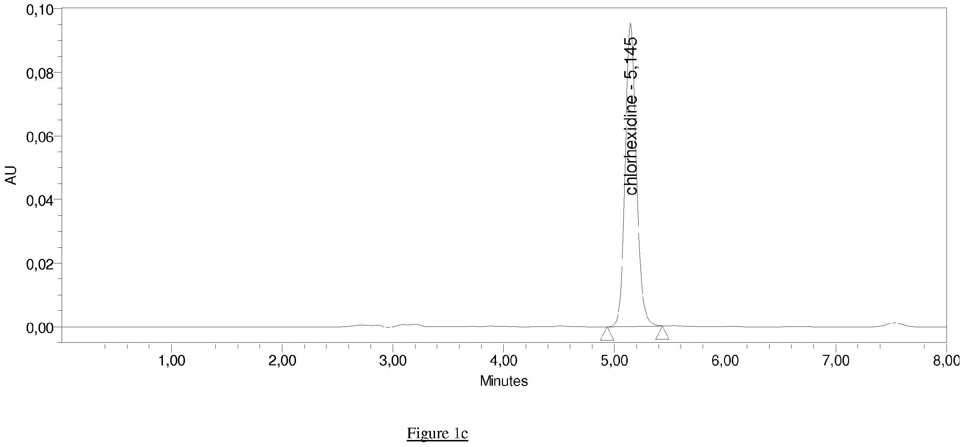

La solution 1 sert de témoin. La quantité de chlorhexidine dans cette solution est stable dans le temps et sensiblement égale à la quantité théorique calculée sur base de la quantité de chlorhexidine contenue dans l'ANIOS ®1916 La coloration de la solution 1 reste inchangée dans le temps, la solution 1 reste limpide et de couleur vert pâle ce qui est consistant avec la couleur verte d'ANIOS ®1916 Les chromatogrammes des figures 1a, 1b et 1c attestent de la très haute pureté de la solution 1 aux différents intervalles de temps mesurés, le pic observé étant celui correspondant à la chlorhexidine.

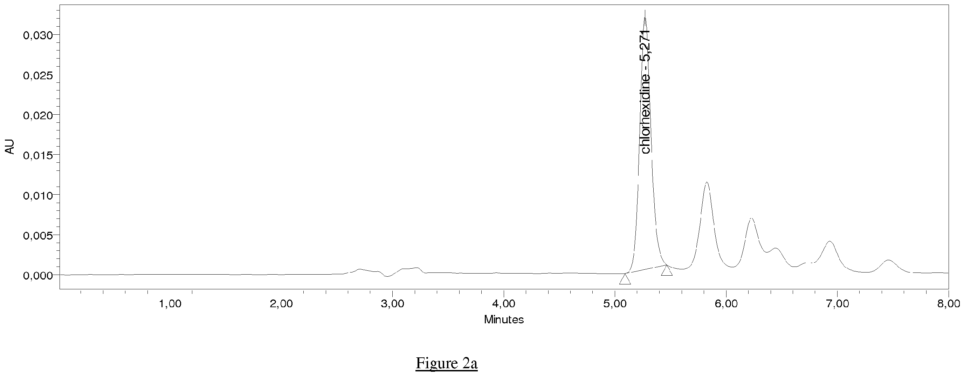

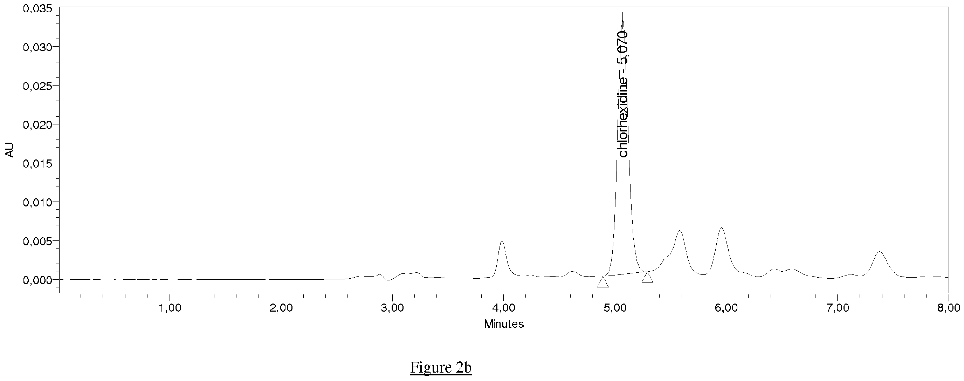

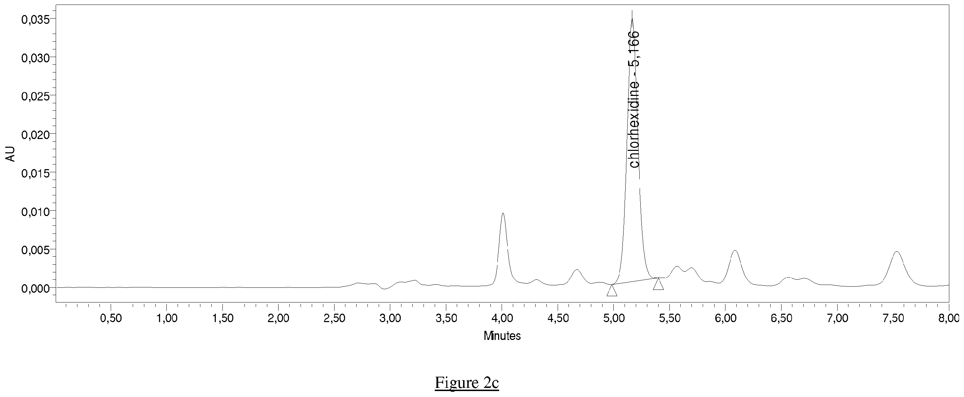

Les résultats obtenus pour la solution 2 montrent qu'il y a une dégradation de la solution dès l'ajout d'hypochlorite de sodium. La quantité de chlorhexidine est diminuée de près de 60 % par rapport à la quantité théorique calculée et la solution prend une couleur marron. Les chromatogrammes des figures 2a, 2b et 2c indiquent la présence de nombreuses impuretés dans la solution.

Ces données expérimentales confirment bien que les solutions contenant de la chlorhexidine ne sont pas stables en présence d'hypochlorite de sodium. Elles deviennent colorées, contiennent des produits de dégradation et ont une teneur en chlorhexidine fortement réduite.

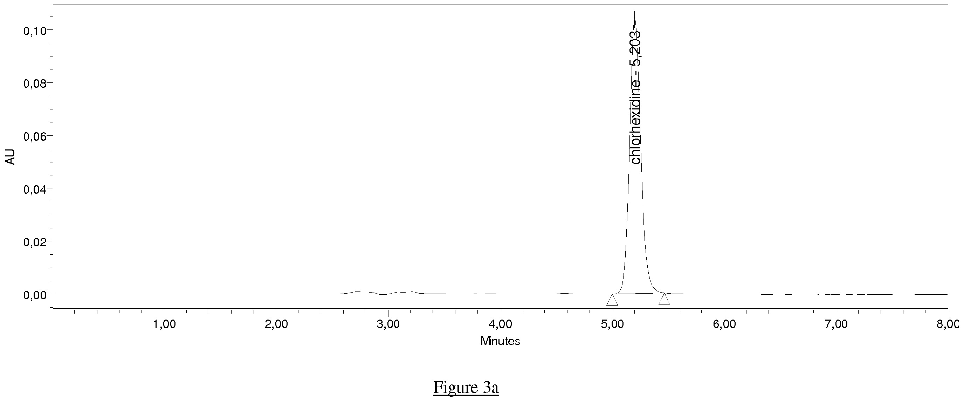

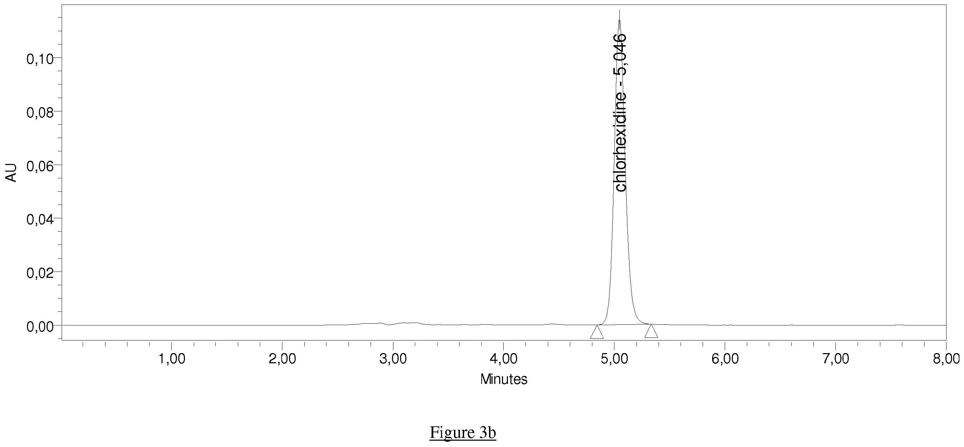

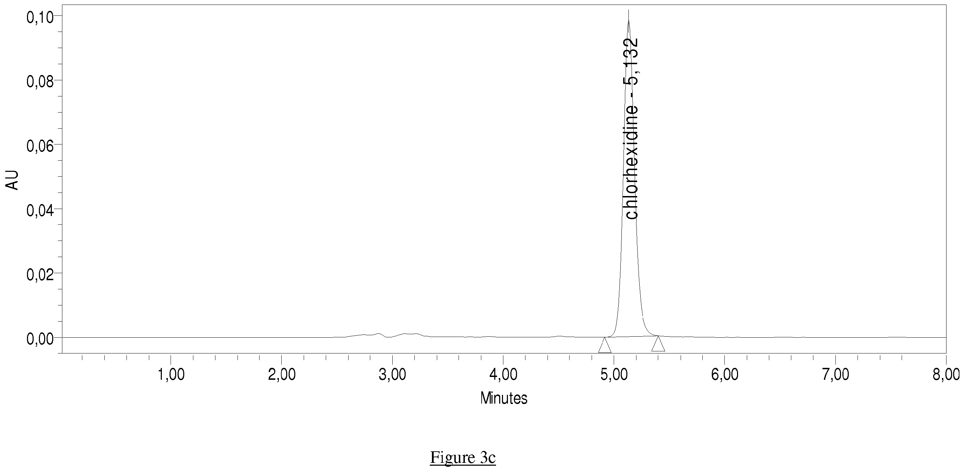

La solution 3 sert de second témoin. De même que pour la solution 1 d'ANIOS ®1916 la quantité de chlorhexidine dans cette solution 3 est stable dans le temps et sensiblement égale à la quantité théorique calculée. De même, la coloration de la solution 3 reste inchangée dans le temps, la solution 3 reste également limpide et de couleur vert pâle. Enfin, les chromatogrammes des figures 3a, 3b et 3c attestent de la très haute pureté de la solution 3 aux différents intervalles de temps mesurés. Les résultats obtenus avec la solution 3 d'ANIOS®1916 contenant du thiosulfate de sodium sont identiques à ceux obtenus avec la solution 1 d'ANIOS ®1916.

En conclusion, l'ajout de thiosulfate de sodium à une solution comprenant de la chlorhexidine n'affecte en rien la stabilité l'aspect et la teneur en chlorhexidine de ladite solution.

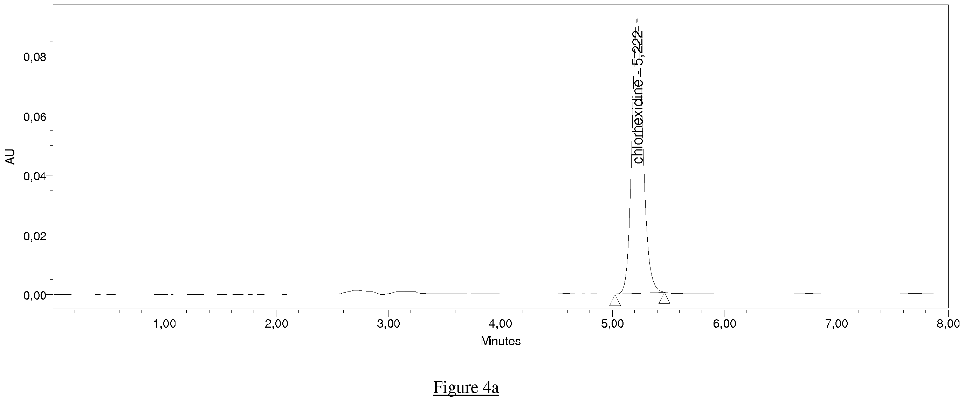

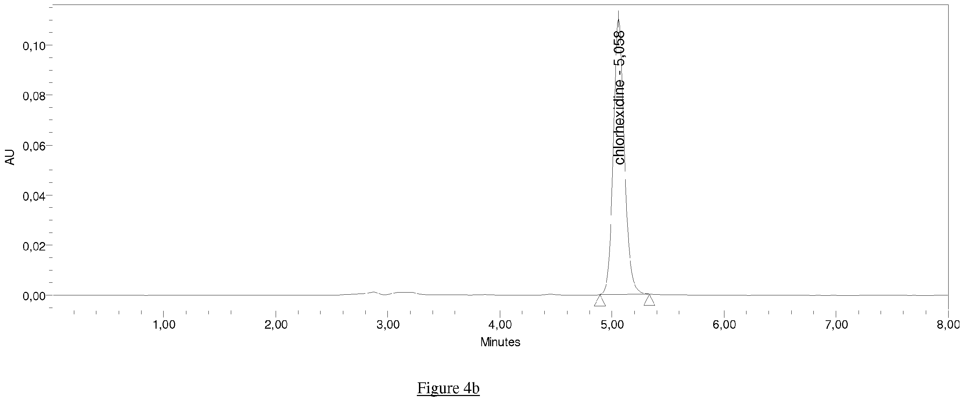

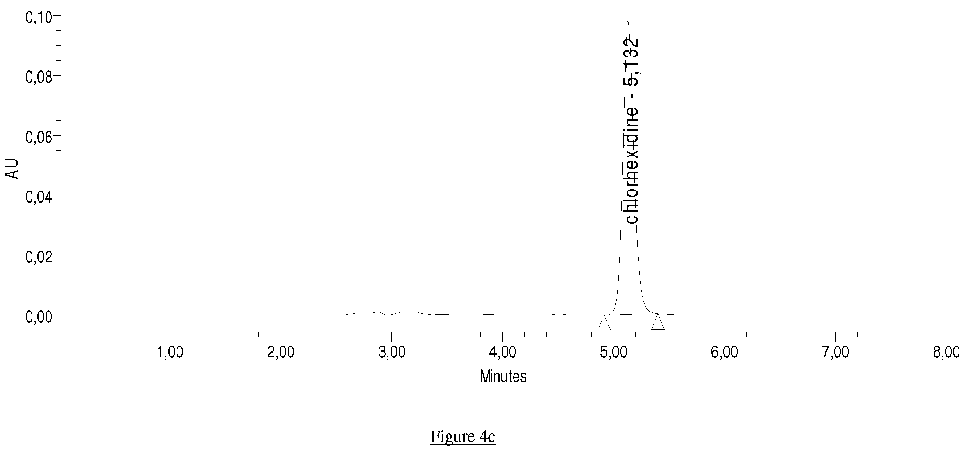

De même que pour les solutions 1 et 3 la quantité de chlorhexidine dans cette solution 4 est stable dans le temps et sensiblement égale à la quantité théorique calculée. De même, la coloration de la solution 4 reste inchangée dans le temps, la solution 4 reste également limpide et de couleur vert pâle. Enfin, les chromatogrammes des figures 4a, 4b et 4c attestent de la très haute pureté de la solution 4 aux différents intervalles de temps mesurés. Ces données expérimentales confirment bien que les solutions contenant de la chlorhexidine et du thiosulfate de sodium sont stables en présence d'hypochlorite de sodium, résultat qui n'avait pas pu être obtenu avec la solution 2 qui ne contenait pas de thiosulfate de sodium.

En conclusion, la composition utilisée selon l'invention permet d'utiliser une solution désinfectante à base de chlorhexidine diluée par une eau contenant de l'hypochlorite de sodium telle que l'eau chaude courante que l'on trouve en milieu hospitalier.

### Exemple 3: étude de l'activité biocide d'une composition selon l'invention.

L'activité biocide des solutions 1, 2 et 4 décrites à l'exemple 2 ont été déterminées selon la norme NF EN 13727 essai en suspension quantitative pour l'évaluation de l'activité bactéricide de formulations nettoyantes désinfectantes. Principe de la NF EN 13727 : une suspension d'essais de bactéries avec substances interférentes est ajoutée à un échantillon du détergent désinfectant dilué dans de l'eau. Le nombre de bactéries de la suspension est ajusté entre 1.5 et 5 x107cfu/ml. Le mélange est maintenu à une température de 20°C +/- 1°C pendant 5 min +/- 10 s. A la fin du temps de contact une partie aliquote est prélevée, l'activité biocide dans cette portion est immédiatement neutralisée par un procédé de dilution/neutralisation. Le nombre de bactéries survivantes dans l'échantillon est déterminé et un facteur de réduction logarithmique calculé. Le produit sera réputé satisfaire la norme NF EN 13727 s'il démontre une réduction supérieur ou égale à 5 log

L'essai a été réalisé avec la souche bactérienne Pseudomonas aeruginosa, avec une concentration en érythrocytes de mouton de 3 mL/L et de la BSA en concentration de 3 g/L pour un temps de contact de 5 minutes. Ces conditions sont représentatives de conditions de saleté.

Cette souche bactérienne est particulièrement pathogène et très résistante. Avec d'autres bactéries à gram-négatif, elle est de plus en plus souvent responsable d'infections nosocomiales. C'est l'une des bactéries les plus difficiles à traiter cliniquement, le taux de mortalité atteint 50% chez les patients vulnérables (immunodéprimés).

L'activité bactéricide est exprimée en log, les résultats sont décrits dans le tableau 4.

**Tableau 4 : Résultats de l'activité bactéricide des solutions 1, 2 et 4**

| **Identification des solutions** | **Dilution dans eau de réseau en % (p/p)** | **Activité** |
|---|---|---|
| **Solution 1** | 0,25% | > 5 log |
| d'ANIOS ^{®}1916 (Lot N011.13S) | | |
| **Solution 2** | 0,25% | 4 log |
| d'ANIOS ^{®}1916 (Lot N011.13S) + 2 ppm de NaOCl | | |
| **Solution 4** | 0,25% | > 5 log |
| d'ANIOS ^{®}1916 (Lot N011.13S) + 0,4 % Na₂S₂O₃ + 2 ppm de NaOCl | | |

L'activité bactéricide de la solution 2 est inférieure à celle de la solution 1. Ce résultat est consistant avec le fait que la solution 2 n'est pas stable et a une quantité de chlorhexidine bien inférieure à celle de la solution 1.

L'activité biocide de la solution 4 est équivalente à celle de la solution 1, la composition selon l'invention a donc un pouvoir bactéricide élevé, ce dernier n'étant en rien affecté par la présence additionnelle de thiosulfate de sodium.

En conclusion, ces exemples démontrent bien que la demanderesse a mis au point une composition nettoyante et/ou désinfectante à base de chlorhexidine utilisable en présence d'hypochlorite de sodium, apportant ainsi une solution innovante au problème technique précédemment mentionné.

## Revendications

1. Utilisation d'une composition nettoyante et/ou désinfectante comprenant de la chlorhexidine ou un sel de chlorhexidine choisi parmi le gluconate de chlorhexidine, le digluconate de chlorhexidine, l'acétate de chlorhexidine, le diacétate de chlorhexidine, le chlorhydrate de chlorhexidine, le dichlorhydrate de chlorhexidine et leurs hydrates, **caractérisée en ce qu'**elle comprend en outre un agent réducteur choisi parmi les thiosulfates minéraux, pour le nettoyage et/ou la désinfection de surfaces dures choisies parmi les sols, les murs, les carrelages, plans de travail, tables, portes, placards et chariots, ou de matériels à usage médical, paramédical, vétérinaire, pharmaceutique ou agro-alimentaire choisis parmi les instruments chirurgicaux, instruments médicaux comprenant les endoscopes, tubes et tuyaux, bassines, la verrerie et les équipements de laboratoire.

2. Utilisation d'une composition selon la revendication 1, **caractérisée en ce qu'**elle comprend du digluconate de chlorhexidine.

3. Utilisation d'une composition selon la revendication 1 ou la revendication 2, **caractérisée en ce que** l'agent réducteur est le thiosulfate de sodium.

4. Utilisation d'une composition selon l'une quelconque des revendications 1 à 3, **caractérisée en ce que** la quantité de chlorhexidine ou de sel de chlorhexidine est comprise entre 1 et 50 mg/g de composition désinfectante.

5. Utilisation d'une composition selon l'une quelconque des revendications 1 à 4, **caractérisée en ce que** la quantité d'agent réducteur est comprise entre 0.5 et 50 mg/g de composition désinfectante.

6. Utilisation d'une composition selon l'une quelconque des revendications 1 à 5, **caractérisée en ce qu'**elle se présente sous forme d'une solution aqueuse.

7. Utilisation d'une composition selon l'une quelconque des revendications 1 à 6, **caractérisée en ce qu'**elle comprend en outre un ou plusieurs agent(s) antimicrobien(s) supplémentaire(s).

8. Utilisation d'une composition selon la revendication 7, **caractérisée en ce que** le ou les agent(s) antimicrobien(s) supplémentaire(s) est/sont choisi(s) dans le groupe constitué par le polyhexaméthylène biguanide (PHMB), le chlorhydrate de polyhexaméthylène biguanide (PHMB-HCl), le chlorure de didécyldiméthylammonium et la N-(3-aminopropyl)-N-dodécylpropane-1,3-diamine.

9. Utilisation d'une composition selon l'une quelconque des revendications 1 à 8, **caractérisée en ce qu'**elle comprend en outre un ou plusieurs excipient(s).

10. Utilisation d'une composition selon la revendication 9, **caractérisée en ce que** le ou les excipient(s) est/sont choisi(s) dans le groupe constitué par les émulsifiants, les tensioactifs de type non-ioniques, cationiques ou encore à caractère amphotère, les hydrotropes, les tiers-solvants de type polyol tels que le glycérol.

## Patentansprüche

1. Verwendung einer Reinigungs- und/oder Desinfektionszusammensetzung, umfassend Chlorhexidin oder ein Chlorhexidinsalz, ausgewählt aus Chlorhexidingluconat, Chlorhexidindigluconat, Chlorhexidinacetat, Chlorhexidindiacetat, Chlorhexidinchlorhydrat, Chlorhexidindichlorhydrat und deren Hydraten, **dadurch gekennzeichnet, dass** diese außerdem ein Reduktionsmittel umfasst, ausgewählt aus mineralischen Thiosulfaten, zur Reinigung und/oder zur Desinfektion von harten Oberflächen, ausgewählt aus Böden, Mauern, Fliesen, Arbeitsflächen, Tischen, Türen, Schränken und Wägen, oder Materialien zur medizinischen, paramedizinischen, veterinären, pharmazeutischen oder agroalimentären Verwendung, ausgewählt aus chirurgischen Instrumenten, medizinischen Instrumenten, umfassend Endoskope, Rohre und Schläuche, Becken, Glaswaren und Laborausrüstungen.

2. Verwendung einer Zusammensetzung nach Anspruch 1, **dadurch gekennzeichnet, dass** diese Chlorhexidindigluconat umfasst.

3. Verwendung einer Zusammensetzung nach Anspruch 1 oder Anspruch 2, **dadurch gekennzeichnet, dass** das Reduktionsmittel Natriumthiosulfat ist.

4. Verwendung einer Zusammensetzung nach einem der Ansprüche 1 bis 3, **dadurch gekennzeichnet, dass** die Menge an Chlorhexidin oder Chlorhexidinsalz zwischen 1 und 50 mg/g der Desinfektionszusammensetzung beträgt.

5. Verwendung einer Zusammensetzung nach einem der Ansprüche 1 bis 4, **dadurch gekennzeichnet, dass** die Menge an Reduktionsmittel zwischen 0,5 und 50 mg/g der Desinfektionszusammensetzung beträgt.

6. Verwendung einer Zusammensetzung nach einem der Ansprüche 1 bis 5, **dadurch gekennzeichnet, dass** diese in Form einer wässrigen Lösung präsentiert wird.

7. Verwendung einer Zusammensetzung nach einem der Ansprüche 1 bis 6, **dadurch gekennzeichnet, dass** diese außerdem ein ergänzendes oder mehrere ergänzende Anti-Mikroben-Mittel umfasst.

8. Verwendung einer Zusammensetzung nach Anspruch 7, **dadurch gekennzeichnet, dass** das oder die ergänzende(n) Anti-Mikroben-Mittel aus der Gruppe bestehend aus Polyhexamethylenbiguanid (PHMB), Polyhexamethylenbiguanidchlorhydrat (PHMB-HCl), Didecyldimethylammoniumchlorid und N-(3-Aminopropyl)-N-dodecylpropan-1,3-diamin ausgewählt ist/sind.

9. Verwendung einer Zusammensetzung nach einem der Ansprüche 1 bis 8, **dadurch gekennzeichnet, dass** diese außerdem einen oder mehrere Exzipienten umfasst.

10. Verwendung einer Zusammensetzung nach Anspruch 9, **dadurch gekennzeichnet, dass** der oder die Exzipient(en) aus der Gruppe bestehend aus Emulgatoren, grenzflächenaktiven Stoffen vom nichtionischen, kationischen Typ oder auch mit amphoterem Charakter, hydrotropen Agentien, Lösungsvermittlern vom Polyol-Typ, wie Glycerin, ausgewählt ist/sind.

## Claims

1. Use of a cleaning and/or disinfecting composition comprising chlorhexidine or a chlorhexidine salt chosen from chlorhexidine gluconate, chlorhexidine digluconate, chlorhexidine acetate, chlorhexidine diacetate, chlorhexidine chlorhydrate, chlorhexidine dichlorhydrate and hydrates thereof, **characterised in that** it further comprises a reducing agent chosen from mineral thiosulphates, for the cleaning and/or disinfecting of hard surfaces chosen from floors, walls, tiles, worktops, tables, doors, cupboards and trolleys, or materials for medical, paramedical, veterinary, pharmaceutical or agro-foods use chosen from surgical instruments, medical instruments comprising endoscopes, tubes and pipes, basins, glassware and laboratory equipment.

2. Use of a composition according to claim 1, **characterised in that** it comprises chlorhexidine digluconate.

3. Use of a composition according to claim 1 or claim 2, **characterised in that** the reducing agent is sodium thiosulphate.

4. Use of a composition according to any of claims 1 to 3, **characterised in that** the quantity of chlorhexidine or of chlorhexidine salt is between 1 and 50 mg/g of disinfecting composition.

5. Use of a composition according to any of claims 1 to 4, **characterised in that** the quantity of reducing agent is between 0.5 and 50 mg/g of disinfecting composition.

6. Use of a composition according to any of claims 1 to 5, **characterised in that** it has the form of an aqueous solution.

7. Use of a composition according to any of claims 1 to 6, **characterised in that** it further comprises one or several additional antimicrobial agents.

8. Use of a composition according to claim 7, **characterised in that** the additional antimicrobial agent or agents are chosen from the group comprising polyhexamethylene biguanide (PHMB), polyhexamethylene biguanide hydrochloride (PHMB-HCl), didecyldimethylammonium chloride and N-(3-aminopropyl)-N-dodecylpropane-1,3-diamine.

9. Use of a composition according to any of claims 1 to 8, **characterised in that** it further comprises one or several excipients.

10. Use of a composition according to claim 9, **characterised in that** the excipient or excipients are chosen from the group comprising emulsifiers, surfactant of the nonionic or cationic type or of an amphoteric nature, hydrotropes, solubilisers of the polyol type such as glycerol.
